(19) Europäisches Patentamt

European Patent Office

Office européen des brevets

(11) **EP 1 467 191 B1**

(12) **EUROPÄISCHE PATENTSCHRIFT**

(45) Veröffentlichungstag und Bekanntmachung des Hinweises auf die Patenterteilung:
**28.06.2006 Patentblatt 2006/26**

(51) Int Cl.:
*G01G 17/00* (2006.01)   *G01G 9/00* (2006.01)

(21) Anmeldenummer: **03008198.8**

(22) Anmeldetag: **08.04.2003**

(54) **Verfahren und Vorrichtung zum Bestimmen der Masse von portionierten Wirkstoffeinheiten**

Method and device for determining the mass of portioned active ingredients units

Procédé et dispositif pour la détermination de la masse des portions d'un agent actif

(84) Benannte Vertragsstaaten:
**AT BE BG CH CY CZ DE DK EE ES FI FR GB GR HU IE IT LI LU MC NL PT RO SE SI SK TR**

(43) Veröffentlichungstag der Anmeldung:
**13.10.2004 Patentblatt 2004/42**

(73) Patentinhaber: **TEWS ELEKTRONIK Dipl.-Ing. Manfred Tews**
**22459 Hamburg (DE)**

(72) Erfinder:
• **Herrmann, Rainer**
**20253 Hamburg (DE)**
• **Schlemm, Udo**
**20259 Hamburg (DE)**
• **Dr.Zaage, Stefan**
**30655 Hannover (DE)**

(74) Vertreter: **Glawe, Delfs, Moll**
**Patentanwälte**
**Rothenbaumchaussee 58**
**20148 Hamburg (DE)**

(56) Entgegenhaltungen:
DE-A- 4 004 119          DE-U- 20 119 344
US-A- 5 515 740          US-A- 5 554 935
US-A- 5 602 485          US-A1- 2002 139 264

• KRASZEWSKI A. W. ET AL.: "Contactless mass determination of arbitrarily shaped objects by microwave resonator measurements" INTERNET, [Online] 12. Oktober 1994 (1994-10-12), XP002252639 Gefunden im Internet: <URL:http://www.nal.usda.gov/ttic/tektran/data/000005/52/0000055265.html> [gefunden am 2003-08-26]

EP 1 467 191 B1

**Beschreibung**

Die Erfindung betrifft ein Verfahren und eine Vorrichtung zum Bestimmen der Masse von portionierten Wirkstoffeinheiten, insbesondere Kapseln, Tabletten oder Dragees, insbesondere der pharmazeutischen Industrie.

Bei portionierten Wirkstoffeinheiten ist es wichtig, zu kontrollieren, ob tatsächlich jede Wirkstoffeinheit die gewünschte Masse hat. Dadurch kann z. B. festgestellt werden, wenn eine Umhüllung nicht vollständig oder überhaupt nicht gefüllt ist. Der Abnehmer wäre zu Recht unzufrieden, wenn die von ihm erworbenen Wirkstoffeinheiten nicht die Menge an Wirkstoffen enthalten, die er erwartet. Dies gilt insbesondere auf dem Gebiet der Pharmazie, wo es auf die genaue Dosierung der Wirkstoffeinheiten ankommt.

In der pharmazeutischen Industrie werden die pharmazeutischen Wirkstoffe zusammen mit bestimmten Füllsubstanzen wie Stärke, Laktose usw. mit speziellen Abfüllmaschinen in Tablettenform gepreßt oder in gelatineartigen Kapseln abgefüllt oder als Dragees ausgebildet. Die entsprechenden Maschinen erreichen hohe Produktionsgeschwindigkeiten mit Abfüllraten von weniger als 50.000 bis zu über 300.000 pro Stunde. Entscheidend für die Wirksamkeit des pharmazeutischen Präparats ist es, daß die Wirkstoffeinheit die nötige Menge an Wirkstoff enthält und dies bei den hohen Abfüllraten auch tatsächlich eingehalten wird. Die Inhaltsmengen der Wirkstoffeinheiten im pharmazeutischen Bereich variieren zwischen 800,mg bei großen Kapseln bis herab zu 50 mg oder bei sehr kleinen Dosen bis zu 5 mg.

Es ist bekannt, die Massen der erzeugten Wirkstoffeinheiten durch Wiegen festzustellen oder zu kontrollieren (DE 198 19 395 C1). Wegen der großen Zahl von Wirkstoffeinheiten pro Zeiteinheit und aufgrund der Tatsache, daß der mechanische Wiegevorgang naturgemäß eine gewisse Zeit benötigt, können entweder nur Stichproben ausgeschleust und gewogen werden, und/oder es muß eine große Anzahl von parallel angeordneten Einrichtungen für die Verwiegung vorgesehen sein. Mit Stichproben kann aber nur der allgemeine Qualitätszustand der produzierten Wirkstoffeinheiten überprüft werden. Eine Einzelüberprüfung ist nicht möglich, so daß Abweichungen von Einzelproben von der mittleren Qualität nicht kontrolliert und ausgeschleust werden können. Andererseits ist die Wiegung jeder einzelnen Wirkstoffeinheit durch parallel angeordnete Wiegevorrichtungen sehr aufwendig. Selbst wenn man z. B. 20 Wiegevorrichtungen verwenden würde, müßte bei den oben angegebenen Raten jede Wiegevorrichtung noch 15.000 wirkstoffeinheiten pro Stunde, also rund 4 pro Sekunde wiegen, was immer noch große technische Schwierigkeiten bedeutet. Die einzelnen Wirkstoffeinheiten müssen bei diesen Produktionsgeschwindigkeiten mechanisch auf dem Wiegegutaufnehmer gestoppt und davor und danach beschleunigt werden. Die rück- und stoßartigen Bewegungen führen zu einer beträchtlichen mechanischen Beanspruchung der Wirkstoffeinheiten. Ferner tritt insbesondere bei mit Umhüllungen versehenen Wirkstoffeinheiten mit kleinen Füllmassen der Nachteil auf, daß die statistischen Schwankungen der Masse der Umhüllung sich als Meßfehler des Füllgewichtes voll niederschlagen. Eine separate zweimalige Verwiegung der gleichen Wirkstoffeinheit ohne und mit Befüllung könnte diesen Fehler ausschließen, was aber im Falle der Verwendung der Wiegetechnik die Probleme noch erhöht. Außerdem ist durch die empfindliche mechanische Führung bei diesen Waagen bei Änderung des Formates der zu wiegenden Wirkstoffeinheiten ein erheblicher mechanischer Umbau von Produktführungen erforderlich.

Es wurde auch versucht, obige Nachteile, die durch die Trägheit der Wiegetechnik hervorgerufen werden, durch kapazitive Meßtechniken zu überwinden (US 4,223,751 A, US 5,602,485 A, DE-OS 29 39 406). Es kann mit dieser Methode bei mit hoher Geschwindigkeit durch den Kondensator bewegten Wirkstoffeinheiten ein masseproportionales elektrisches Signal nur dann bestimmt werden, wenn der Feuchtegehalt der Wirkstoffeinheiten und des Füllmaterials exakt konstant bleibt. Durch leichte Fluktuationen des Wassergehaltes des Wirkstoffs in den Wirkstoffeinheiten wird infolge der großen Dielektrizitätskonstante des Wassers eine überproportionale Änderung des Massesignals erzeugt. Eine kleine Veränderung der Feuchte erzeugt somit eine große Abweichung des Massesignals von der tatsächlichen Masse.

Bei einem Verfahren zur Massebestimmung von beliebig geformten dielektrischen Gegenständen mithilfe eines Mikrowellenresonators (US 5 554 935A) wird ein Transmissionskoeffizient gemessen. Die dort erwähnte Messung der Feuchte dürfte aber zumindestens bei den in erster Linie erwähnten Kunststoffteilchen im Gegensatz zu portionierten Wirkstoffeinheiten, insbesondere Kapseln, Tabletten oder Dragees nur eine untergeordnete Rolle spielen.

Die Aufgabe der Erfindung besteht in der Schaffung eines Verfahrens und einer Vorrichtung der eingangs genannten Art, mit dem die Masse der Wirkstoffeinheiten zuverlässig, genau und schnell ohne übermäßigen Aufwand bestimmt werden kann.

Die erfindungsgemäße Lösung besteht bei einem Verfahren der eingangs genannten Art darin, daß die Wirkstoffeinheiten durch einen Mikrowellenresonator geleitet werden und aus der Verschiebung der Resonanzfrequenz und der Verbreiterung der Resonanzkurve gemäß Anspruch 1 die Masse bestimmt wird.

Die Erfindung kann dabei Gebrauch machen von einem Verfahren, mit dem die Masse und/oder die Feuchtigkeit eines Materials bestimmt werden kann (EP 0 468 023 B1). Es ist das Verdienst der Erfindung, erkannt zu haben, daß das Prinzip der Messung mit Mikrowellen auch für die Bestimmung der Masse von portionierten Wirkstoffeinheiten verwendet werden kann. Die Messung mit Mikrowellen hat dabei den Vorteil, daß sie sehr schnell ist. Entsprechend schnell wird sich die Verschiebung der Resonanzfrequenz oder die Breite der Resonanzkurve ändern, wenn Teilchen durch den Mikrowellenresonator hindurchgeleitet werden. Die Erfindung hat hier erkannt, daß das erzeugte Signal so ausgewertet werden kann, daß trotzdem die Masse der einzelnen Wirkstoffeinheiten bestimmt werden kann. Dies geschieht z. B. durch Bestimmung jeweils des Maximums der Verschiebung der Resonanzfrequenz und/oder der Breite der Resonanzkurve beim Durchgang einer Wirkstoffeinheit.

Das genannte bekannte Verfahren (EP 0 468 023 B1) und ähnliche bekannte Verfahren ermöglichen die gleichzeitige Messung zweier Eigenschaften des dielektrischen Produktes, das sich gerade im Meßfeld des Resonators befindet:

> 1. Die Veränderung A der Resonanzfrequenz gegenüber der Resonanzfrequenz des leeren Zustands, ein Effekt, der proportional zur Dielektrizitätskonstanten (Realteil) der Wirkstoffeinheit und daher ihrer Masse ist.

> 2. Die Zunahme B der Halbwertsbreite der Resonanzkurve gegenüber derjenigen des leeren Zustands, ein Effekt, der nicht nur proportional zur Masse der Wirkstoffeinheit, sondern auch zu Umwandlung von Mikrowellenenergie in Wärme durch das in Produkt enthaltene Wasser ist (Imaginärteil der Dielektrizitätskonstanten).

Bei einer vorteilhaften Ausführungsform wird zusätzlich zur Masse auch die Feuchte bestimmt, um so Qualitätsschwankungen besser feststellen zu können.

Beim Bestimmen der Masse von portionierten Wirkstoffeinheiten, die aus einer Umhüllung und dem darin enthaltenen Wirkstoff bestehen, kann bei einer vorteilhaften Ausführungsform vorgesehen werden, daß die Masse der Umhüllung ohne Wirkstoff und anschließend die Gesamtmasse der Wirkstoffeinheit nach der Befüllung mit dem Wirkstoff bestimmt wird. Dadurch kann sichergestellt werden, daß auch bei ungleichförmiger Masse der Umhüllungen immer die gleiche Wirkstoffmenge in die Umhüllungen enthalten ist.

Man könnte vorsehen, daß die leeren Umhüllungen nach Masse sortiert und anschließend chargenweise mit gleicher Masse gefüllt werden und die Gesamtmasse bestimmt wird. Zweckmäßiger ist es aber, wenn die Masse der Umhüllung unmittelbar vor der Befüllung bestimmt wird. Die einzelne Wirkstoffeinheit, deren Geschwindigkeit bekannt ist, kann dann elektronisch von der ersten Meßstelle zur zweiten Meßstelle verfolgt werden, so daß das Leergewicht und das befüllte Gewicht jeder einzelnen Wirkstoffeinheit miteinander korreliert werden können.

Zweckmäßigerweise werden die Messungen mit Mikrowellenfrequenzen von 1 bis 60 GHz, insbesondere 2 bis 30 GHz durchgeführt.

Wird mit dem Verfahren eine Abweichung der Wirkstoffmasse von einem vorgegebenen Toleranzbereich festgestellt, so kann die Wirkstoffkapsel mit an sich bekannten Verfahren wie z. B. eine mechanische Weiche oder einen Luftstoß ausgeschleust werden.

Eine weitere Lösung der Aufgabe besteht in der Schaffung einer Vorrichtung, die einen Mikrowellengenerator, einen Mikrowellenresonator, eine Einrichtung zum Führen der Wirkstoffeinheit durch den Mikrowellenresonator, eine Meß- und Auswertungselektronik zum Bestimmen der Masse und eine Einrichtung zum Ausschleusen einzelner Wirkstoffeinheiten aufweist.

Die Einrichtungen zum Führen der Wirkstoffeinheiten können eine Röhre aufweisen, durch die die Wirkstoffeinheiten mit einem Luftstrom befördert werden. Andererseits kann vorgesehen werden, daß ein Endlosband mit Vertiefungen vorgesehen ist, in die die Wirkstoffeinheiten eingelegt werden und das durch den Resonator hindurchbewegt wird.

Eine weitere vorteilhafte Alternative besteht in einer kreisrunden Scheibe, an deren Umfang die Wirkstoffeinheiten mit Hilfe von Unterdruck festgehalten werden. Nach dem Meßvorgang können die Teilchen dann durch einen leichten Überdruck schnell von der Scheibe entfernt werden.

Wird ein Träger in Form eines Endlosbandes oder einer Scheibe verwendet, so trägt dessen Masse zur Verschiebung und Verbreiterung der Resonanzkurve bei. Dieser Beitrag muß vorher bestimmt werden. Falls das Band oder die Scheibe nicht vollständig gleichmäßig ist, müssen dabei die Ungleichmäßigkeiten mit dem Ort auf dem Band oder der Scheibe korreliert werden, so daß für verschiedene Wirkstoffeinheiten unterschiedliche Korrekturen vorgenommen werden. Alternativ oder zusätzlich kann dieser Massenbeitrag aber auch zwischen einzelnen Wirkstoffeinheiten an einer Stelle gemessen werden, wo sich keine Wirkstoffeinheit befindet. Dies eröffnet die Möglichkeit, allmähliche Änderungen durch z. B. Verschmutzung oder Temperaturänderungen festzustellen und zu kompensieren.

Eine zweckmäßige Vorrichtung zur Bestimmung der Masse von Wirkstoffeinheiten, die aus einer Umhüllung und dem darin enthaltenen Wirkstoff bestehen, zeichnet sich dadurch aus, daß die einen zweiten Mikrowellenresonator mit Meß- und Auswertungselektronik zum Bestimmen der Masse der Wirkstoffeinheiten vor der Befüllung aufweist.

Die Erfindung wird im folgenden anhand von vorteilhaften Ausführungsformen unter Bezugnahme auf die beigefügten Zeichnungen beispielsweise beschrieben. Es zeigen:

Figur 1       den Einfluß unterschiedlicher in der Pharmaindustrie üblicher Kapselgrößen auf die Verschiebung der Resonanzfrequenz und die Verbreiterung der Resonanzkurve eines Mikrowellenresonators;

Figur 2       die Abhängigkeit des Mikrowellenmassewertes vom Gewicht der Kapseln;

Figur 3      die Änderung der Differenz des mit Mikrowellen gemessenen Massewertes zum entsprechenden Massewert der leeren Umhüllungen für unterschiedliche Kapselgewichte;

Figur 4      die Abhängigkeit des gemessenen Massewertes von der Füllmenge bei sehr kleinen Füllmengen;

Figur 5      die Anordnung eines Mikrowellenresonators am Ausgang einer Maschine zur Herstellung/Befüllung von Wirkstoffeinheiten;

Figur 6      das Mikrowellensignal beim Durchgang einer Wirkstoffeinheit durch den Resonator;

Figur 7      eine Ausführungsform eines Mikrowellenresonators;

Figur 8      eine andere Ausführungsform eines Mikrowellenresonators;

Figur 9      eine noch andere Ausführungsform eines Resonators mit einer unterschiedlichen Führung für die Wirkstoffeinheiten; und

Figur 10      noch eine andere Anordnung zur Messung des Masse der Wirkstoffeinheiten.

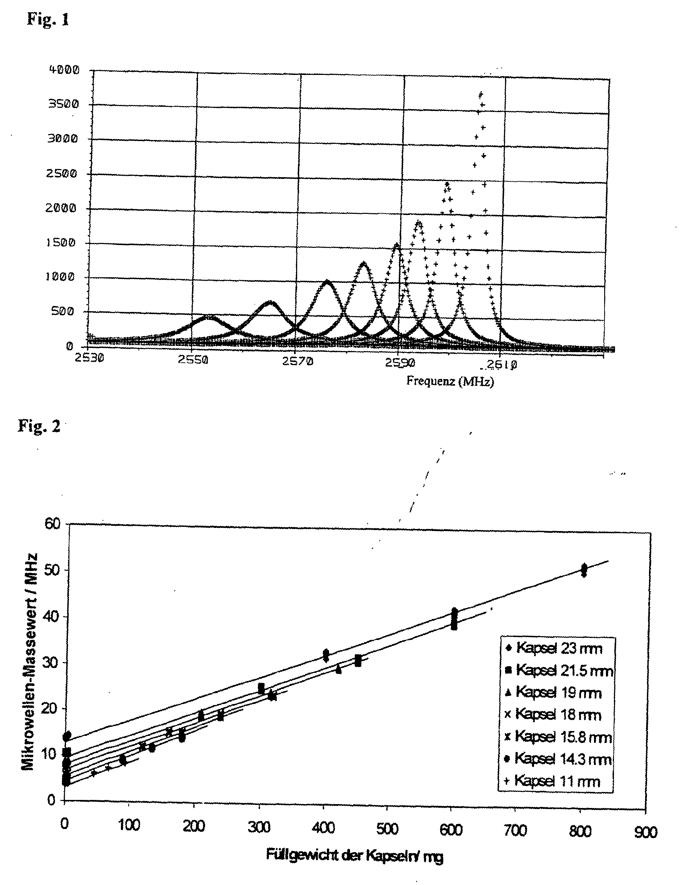

Die Wirkung auf die Mikrowellenresonanz durch die unterschiedlichen in der Pharmaindustrie üblichen Kapselgrößen zeigt Figur 1. Je größer die Masse der Kapsel ist, um so größer ist die Verschiebung A der Resonanzfrequenz gegenüber der ganz rechts angeordneten Resonanzkurve des leeren Resonators. Um so größer ist auch die Dämpfung und Verbreiterung B der Resonanzkurve, hervorgerufen durch das im Pulver der Kapsel enthaltene Wasser. Der Quotient beider Größen, F=B/A ist unabhängig von der Masse der Kapsel und ein Maß für den prozentualen Anteil des Wassers. Dies gilt natürlich auch für Tabletten, Dragees oder andere Wirkstoffeinheiten. Damit bietet das Resonatorverfahren nicht nur eine Masse proportionale Meßgröße A (oder B) zur Massebestimmung, sondern auch eine Größe F zur Feuchtebestimmung und gleichzeitig zur Kompensation des Feuchteeinflusses auf das Massesignal. Eine einfache, aber wirkungsvolle Methode besteht z. B. darin, die Masse M der Wirkstoffeinheit gegenüber einer direkten Massemessung mit einer Waage bei zwei unterschiedlichen Feuchten zu kalibrieren und damit die Koeffizienten k1 und k2 folgender Gleichung zu bestimmen:

$$M = k1 * (1 + k2 * F) * A$$

In der Regel ist k2 eine für die betreffende Wirkstoffeinheit typische Konstante und kann im Speicher der CPU der Füllmaschine abgelegt und unter dem entsprechenden Stichwort für das Füllmaterial (z. B. Stärke oder Laktose usw.) abgerufen werden. Zur einfachen Schnellkalibration genügt dann die Bestimmung der Konstanten k1 als Ein-Punkt-Kalibration. Dies kann auch online dadurch geschehen, daß die stichprobenartige Kontrolle, die oftmals wegen der Eichfähigkeit der Wägemethode vom Pharmahersteller vorgeschrieben wird, beibehalten wird, und somit durch Vergleich zwischen dem Waagenwert und dem Mikrowellenwert die Konstante k1 online abgeglichen wird.

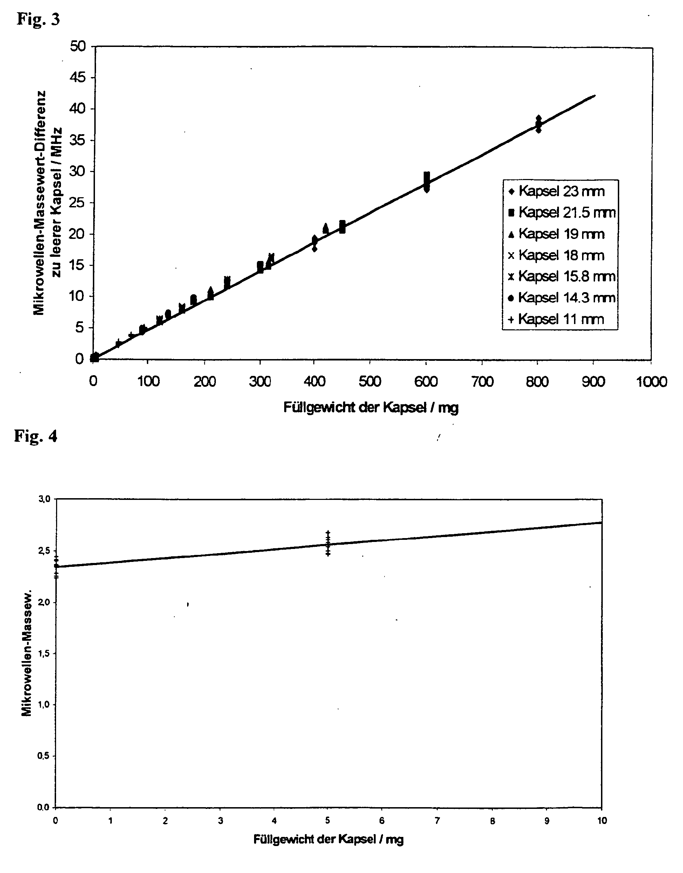

Figur 2 zeigt, wie präzise der Mikrowellenmassewert die unterschiedlichen Kapselfüllungen für alle möglichen Kapselgrößen zwischen Volumina von 23*8,5 mm bis herab zu 11*4,5 mm repräsentiert, wobei jede Kapselsorte mit einer definierten Füllung zehnmal vermessen wurde. Dabei sind die Schwankungen der Meßwerte der einzelnen Kapseln auf die statistischen Schwankungen der Massen der leeren Gelatinekapseln zurückzuführen. Die Mikrowellenmesswerte für die verschiedenen Kapselgrößen liegen auf parallel verschobenen Geraden, wobei die Lage durch den Mikrowelleneffekt der leeren Kapseln bestimmt wird. Dies zeigt Figur 3, in der die Änderungen des Mikrowelleneffektes gegenüber dem leeren Resonator aufgetragen ist. Die Beziehung zwischen der Änderung des Mikrowellen-Massemesswertes gegenüber dem der leeren Kapsel und der Füllmasse der Kapsel läßt sich somit in guter Näherung als eine einzige Gerade darstellen.

Figur 4 zeigt, daß selbst bei kleinsten Kapselfüllmengen von nur 5 mg ein deutlicher Anstieg des Signals gegenüber den leeren Kapseln zu erkennen ist. Allerdings ist beim Vergleich der Streubreite der Mikrowellen-Messignale der leeren und der gefüllten Kapsel zu erkennen, daß die Massevariationen innerhalb der leeren Kapsel bereits die Größenordnung der Menge des zu messenden Füllmaterials haben. In diesem Falle ist zur Erlangung einer ausreichenden Messpräzision entweder eine Zweifachmessung der Kapsel mit zwei Mikrowellenresonatoren erforderlich (einer vor und einer nach der Befüllung), oder aber eine Vorsortierung der Kapseln, so daß nur Kapseln mit identischer Masse eingesetzt werden.

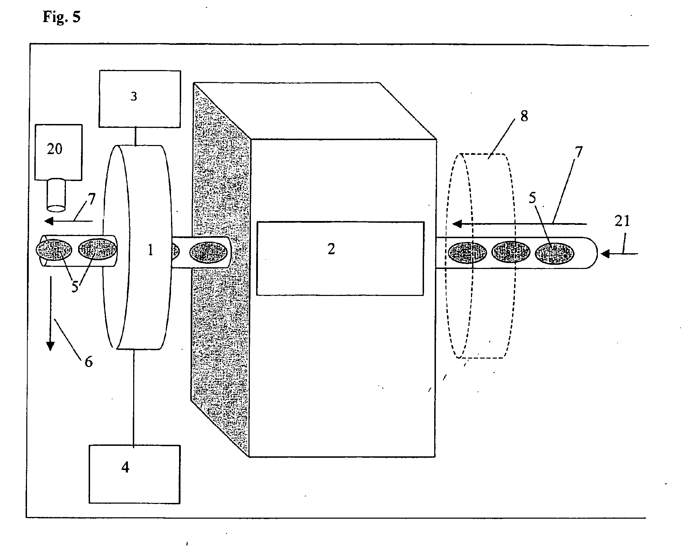

In Figur 5 ist die Anordnung eines Mikrowellenresonators 1 am Ausgang einer Maschine zur Befüllung von Kapseln dargestellt. Der Mikrowellenresonator 1 wird von einem Mikrowellengenerator, der bei 3 schematisch dargestellt ist, mit Mikrowellenenergie gespeist. Die austretende Mikrowellenenergie wird dann durch eine bei 4 angedeutete Schaltung gemessen und mit einer darin enthaltenen Elektronik ausgewertet. Ergibt die Messung einer Kapsel 5, daß ihre Masse nicht innerhalb vorgegebener Toleranzgrenze liegt, so wird die entsprechende Kapsel 5 durch eine bei 20 angedeutete Auswerfeinrichtung mit Hilfe eines Luftstoßes oder z. B. in Form einer Weiche in Richtung des Pfeiles 6 aus dem Strom der Kapseln 5 herausgelenkt, der normalerweise in Richtung des Pfeiles 7 verläuft, wobei die Kapseln durch einen Luftstrom transportiert werden, der bei 21 angedeutet ist. Über entsprechende Weichen oder Luftimpulse kann auch eine Sortierung der Kapseln nach unterschiedlichen Massen bzw. Feuchten in unterschiedlichen Behältern durchgeführt werden. Dabei kann die Mikrowellenresonator-Meßeinheit 1, 3, 4 entweder zusammen mit dem Auswurfmechanismus in die Maschine 2 selbst integriert werden oder aber als eigenständige Einheit neben der Maschine 2 positioniert werden, die die Kapseln aus einem Zwischenpuffer entnimmt .

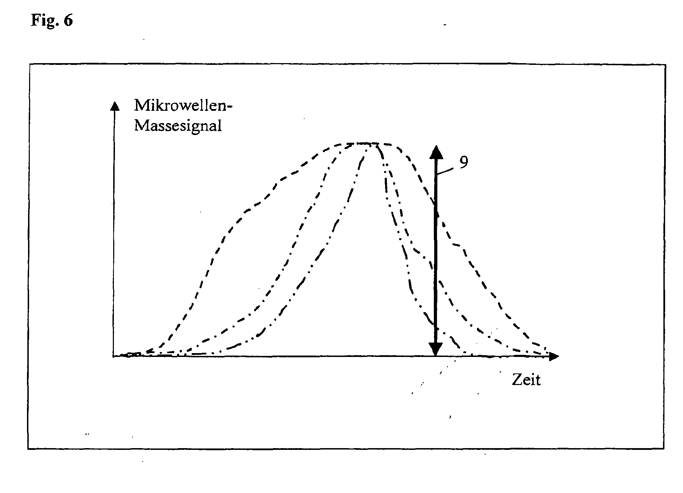

Im besonderen Fall sehr kleiner Füllmassen in Kapseln 5 bei denen der Schwankungsbereich der leeren Kapselnmasse im Bereich des Kapselinhaltes liegt, ist es sinnvoll, eine Kapselfüllanlage mit zwei Mikrowellenresonatoren auszustatten. Zusätzlich zum Mikrowellenresonator 1 am Ausgang der Maschine 2 kann ein weiterer Mikrowellenresonator 8, der in Figur 6 gestrichelt dargestellt ist, am Eingang der Maschine 2 vorgesehen sein. Dieser ist ebenfalls mit einem Mikrowellengenerator 3 und einer Meß- und Auswerteschaltung 4 versehen, die aber in Figur 5 nicht gezeigt sind. Wenn der Steuerrechner der Maschine 2 den Weg jeder einzelnen Kapsel verfolgt, kann er die Differenz der entsprechenden vollen und leeren Kapselmassen bestimmen und den Inhalt klassifizieren bzw. die Kapseln sortieren.

Die besondere Anforderung an das Mikrowellenmessfeld zur Wirkstoffeinheit-Massemessung besteht darin, daß es homogen sein muß über die gesamte Zone, durch die sich die Wirkstoffeinheit bewegt und die für den Messwert bestimmend ist. Nur dann ist gewährleistet, daß das masseproportionale Messsignal nicht von der Art der Bewegung und Stellung der Wirkstoffeinheit im Messfeld bzw. der Art der Verteilung der Pulvermasse innerhalb der Kapsel abhängt. Auf diese Art erzeugt eine sich bewegende Wirkstoffeinheit mit einer bestimmten Gesamtmasse unabhängig von der Art der Bewegung oder Verteilung der Masse innerhalb der Probe einen zeitlichen Signalverlauf, der zwar im einzelnen von der konkreten Bewegungsart abhängt, aber einen identischen, durch die Probenmasse definierten Maximumwert aufweist. Denn während der Bewegung der Wirkstoffeinheit durch den Resonator nehmen die masseproportionalen Werte A und B zu, erreichen ein Maximum, wenn sich die gesamte Wirkstoffeinheit im elektrischen Messfeld des Resonators befindet. Das über die Probe konstante Mikrowellenfeld wirkt wie ein Intergrationsglied und erzeugt einen Maximalwert 9, der eindeutig ein Maß für die Masse der Wirkstoffeinheit ist, wie dies in Figur 6 gezeigt ist. Die drei Kurven zeigen dabei die Signale identischer Wirkstoffeinheiten, die sich aufgrund unterschiedlicher Bewegungsverläufe beim Durchgang durch den Resonator unterscheiden. Sie weisen aber denselben Maximalwert 9 auf, der ein Maß für die Masse der Wirkstoffeinheit ist.

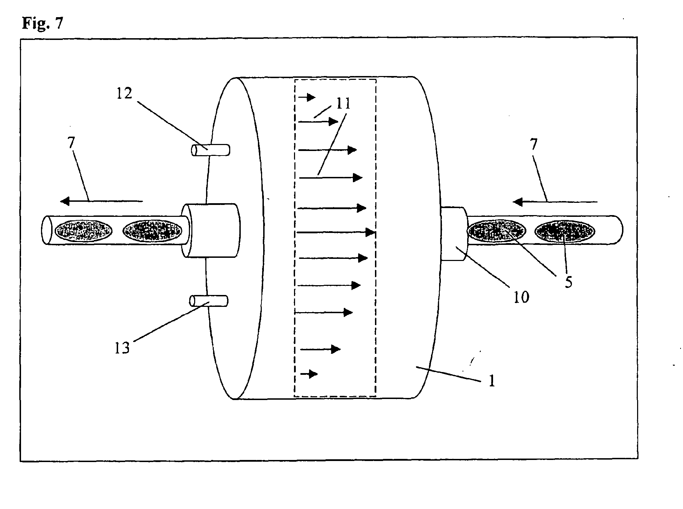

Eine Ausführungsform für einen Mikrowellenresonator 1 zeigt die Figur 7. Ein Resonator 1 im E010-Grundmodus ist mit einem dielektrischen Probenrohr 10 versehen, dessen Innendurchmesser von 10 mm etwas größer als der größte zu erwartende Wirkstoffeinheitdurchmesser ist. Die elektrische Feldstärke 11 ist sowohl in Bewegungsrichtung als auch in der dazu senkrechten Ebene im Bereich des Probenrohres konstant, wenn sich die Probe in der Mitte des Resonators befindet, weshalb dieser Resonatortyp zur Massebestimmung in Wirkstoffeinheiten formatunabhängig mit Probendurchmessern zwischen 9.5 mm und 2 mm gut geeignet ist. Bei 12 und 13 sind Anschlüsse gezeigt, über die die Mikrowellen in den Resonator 1 und aus demselben herausgekoppelt werden.

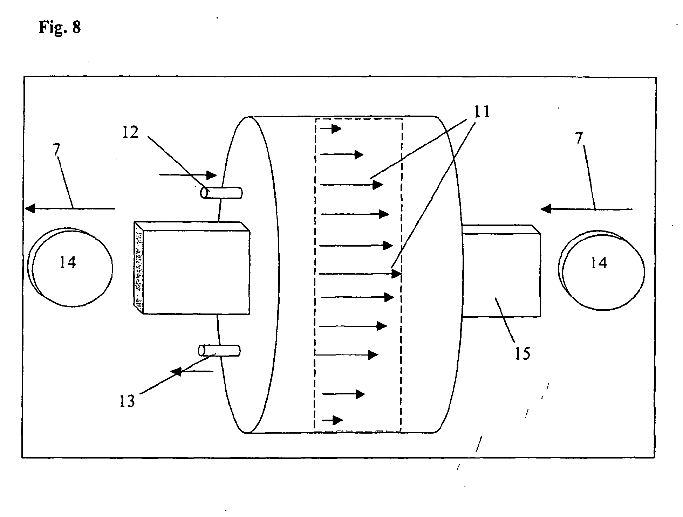

Figur 8 zeigt einen Resonatortyp zur Vermessung von Tabletten 14 mit relativ großen Durchmessern (z. B. Brausetabletten), wobei durch die rechteckige Probenführung 15 eine Integration über die gesamte Tablette möglich ist, wenn die Tablette sich in der Resonatormitte befindet. Auch dieser Resonator wird in Resonanz im E010-Modus angeregt, wobei die leichte Inhomogenität im Feldlinienbild bei sehr großen Probeführungs-Dimensionen um so weniger ins Gewicht fällt, je niedriger die Meßfrequenz, d. h. je größer der Resonatordurchmesser gewählt wird. Begrenzt wird diese Herabsetzung der Meßfrequenz durch die Maßgabe, daß bei Frequenzen unterhalb von 1 GHz die Verluste aufgrund von Ionenleitfähigkeitseffekten gegenüber den Wasserdipoverlusten nicht mehr vernachlässigt werden können und damit eine zuverlässige Feuchtemessung nicht mehr möglich ist.

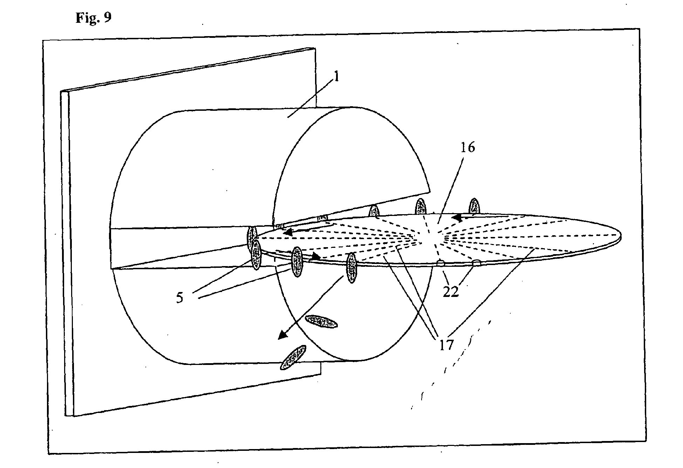

Figur 9 zeigt einen Resonator 1 vom Typ eines einseitig geöffneten Gabelresonators, durch den die Wirkstoffeinheiten in senkrechter Position geführt werden können, wobei sie über eine radförmige Kunststofführung 16 und Unterdruckansaugung 17 in ihrer Position gehalten werden. Der Mikrowellenresonator 1 wird dabei in einer vom zylindrischen Resonator bekannten E010-Resonanz angeregt, wobei die Wandflächen so aufgetrennt wurden, daß die Wandströme nicht beeinträchtigt werden. Sieht man genügend stark ausgebildete Seitenwände vor, so daß innerhalb dieses Bereiches nach außen ein exponentieller Feldabfall möglich ist, ist dieser Resonator trotz großer Öffnungen vollkommen abstrahlungsfrei und besitzt quer zur Bewegungsrichtung der Wirkstoffeinheiten ein sehr homogenes Messfeld. Damit sind die Voraussetzungen für eine präzise Masse- und Feuchtemessung gegeben. Ein weiterer Vorteil dieser Anordnung des Resonators ist, daß zu jedem Zeitpunkt die exakte Position der Wirkstoffeinheiten bekannt ist, so daß die anschließende Behandlung und Sortierung der Wirkstoffeinheiten nach den Ergebnissen der Messung leicht möglich ist. Um die exakten Mikrowellenmesswerte der Wirkstoffeinheiten zu erhalten, müssen vorher die Mikrowelleneffekte Ao und Bo der Wirkstoffeinheitführung im Zustand ohne Wirkstoffeinheit vermessen werden. Dies kann z. B. in einer separaten Tarierungsprozedur zu Beginn des eigentlichen Produktionsprozesses nach Einschalten der Maschine im Leerlauf geschehen.

Die eigentlichen Wirkstoffeinheit-Messwerte ergeben sich dann als Differenzwerte aus den aktuellen Messwerten A und B und den für die einzelnen Positionen gültigen Messwerten der Wirkstoffeinheitführung Ao und Bo. Sieht man in dem Führungsrad eine oder mehrere Positionen vor, an denen der Mikrowelleneffekt alleine durch das Führungsrad ohne Wirkstoffeinheit gemessen werden kann (z. B. die in der Fig. 9 mit 22 bezeichneten Positionen), ist durch Vergleich mit den entsprechenden Werten der Anfangstarierung eine Online-Tarierung möglich, so daß auch im vielstündigen Non-Stop-Betrieb der Effekt von Temperaturänderungen oder Resonator-Verschmutzungen automatisch kompensiert werden kann, ohne den Prozess anzuhalten. Damit wird eine präzise Masse- und Feuchtemessung auch bei Veränderung der Temperatur und Verschmutzung der Messzone garantiert. Bei Änderung des Formates der Wirkstoffeinheiten ist lediglich ein Austausch des Führungsrades 16 mit den geeigneten Passformen 22 für die neuen Wirkstoffeinheiten vorzunehmen.

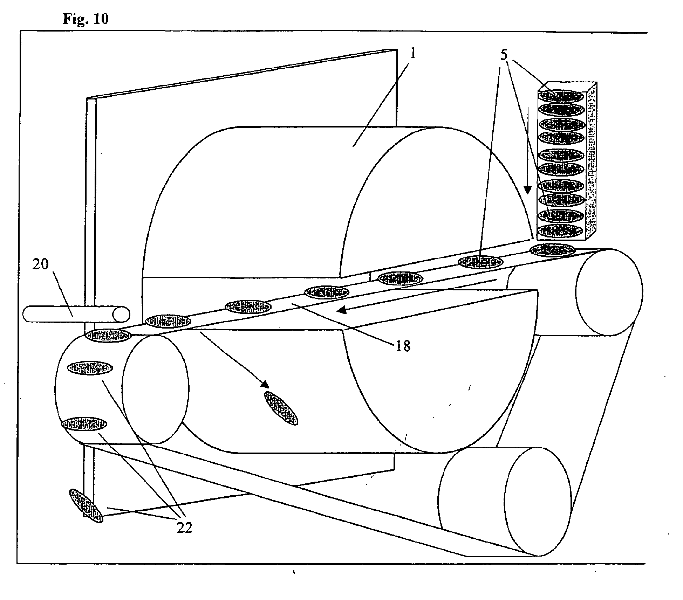

Figur 10 zeigt ein weiteres Ausführungsbeispiel einer Wirkstoffeinheit 18, wobei sich als Resonatortyp wiederum ein einseitig geöffneter oder beidseitig geschlossener Gabel-Resonator 1 mit einem sehr homogenen Querfeld zur präzisen Masse- und Feuchtemessung eignet. Da die Wirkstoffeinheiten 5 in Vertiefungen 22 der Führung 18 abgelegt werden, ist die exakte Position der Wirkstoffeinheiten genau bekannt, so daß auch hier eine leichte weitere Sortierung der Wirkstoffeinheiten möglich ist. Wie in Figur 9 muß der Mikrowelleneinfluß der Wirkstoffeinheitführung 18 durch eine Anfangstarierung sowie seine Änderung durch eine punktweise Kontrolle der Führung online sichergestellt werden. Formatänderungen der Wirkstoffeinheiten erfordern auch hier einen Austausch der Führung 18, die hier als Endlosband ausgeführt ist, was aber einfach durchgeführt werden kann.

## Patentansprüche

1. Verfahren zum Bestimmen der Masse von portionierten Wirkstoffeinheiten, insbesondere Kapseln, Tabletten oder Dragees, bei dem die Wirkstoffeinheiten durch einen Mikrowellenresonator geleitet werden und aus der Verschiebung A der Resonanzfrequenz und der Verbreiterung B der Resonanzkurve die Masse M unter Kompensation des Feuchteeinflusses durch eine mathematische Verknüpfung der beiden Messgrößen bestimmt wird, die aus einer linearen Entwicklung von M nach A besteht, wobei jeder der Koeffizienten eine lineare Entwicklung nach F darstellt, wobei F=B/A ist und die Masse mit Hilfe der Beziehung

$$M = k1*(1+k2*F)*A$$

bestimmt wird, wobei k1 und k2 Konstanten sind, wobei die Konstante k2 von der untersuchten Wirkstoffeinheit abhängt.

2. Verfahren nach Anspruch 1, **dadurch gekennzeichnet, dass** zusätzlich die Feuchte bestimmt wird.

3. Verfahren nach Anspruch 1 oder 2, zum Bestimmen der Masse von portionierten Wirkstoffeinheiten, die aus einer Umhüllung und dem darin enthaltenen Wirkstoff bestehen, **dadurch gekennzeichnet, dass** die Masse der Umhüllung ohne Wirkstoff und anschließend die Gesamtmasse der Wirkstoffeinheit nach der Befüllung mit dem Wirkstoff bestimmt wird.

4. Verfahren nach Anspruch 3, **dadurch gekennzeichnet, dass** die leeren Umhüllungen nach Masse sortiert und anschließend chargenweise befüllt und die Gesamtmasse bestimmt wird.

5. Verfahren nach Anspruch 3, **dadurch gekennzeichnet, dass** die Masse der Umhüllung unmittelbar vor der Befüllung bestimmt wird.

6. Verfahren nach einem der Ansprüche 1 bis 5, **dadurch gekennzeichnet, dass** Mikrowellen mit Frequenzen von 1 bis 60 GHz, insbesondere 2 bis 30 GHz verwendet werden.

7. Vorrichtung zum Bestimmen der Masse von portionierten Wirkstoffeinheiten (5, 14), insbesondere Kapseln, Tabletten oder Dragees, die einen Mikrowellengenerator (3), einen Mikrowellenresonator (1), eine Einrichtung (10, 15, 16) zum Führen der Wirkstoffeinheiten (5, 14) durch den Mikrowellenresonator (1), eine Mess- und Auswertungselektronik (4) zur Bestimmung der Masse aus der Verschiebung A der Resonanzfrequenz und der Verbreitung B der Resonanzkurve und eine Einrichtung zum Ausschleusen einzelner Wirkstoffeinheiten (5, 14) aufweist.

8. Vorrichtung nach Anspruch 7, **dadurch gekennzeichnet, dass** die Einrichtung zum Führen der Wirkstopfeinheiten eine Röhre (10, 15) aufweist, durch die die Wirkstoffeinheiten (5, 14) mit einem Luftstrom (21) gefördert werden.

9. Vorrichtung nach Anspruch 7, **dadurch gekennzeichnet, dass** die Einrichtung zum Führen der Wirkstoffeinheiten ein Endlosband (18) mit Vertiefungen (22) aufweist, in die die Wirkstoffeinheiten eingelegt werden.

10. Vorrichtung nach Anspruch 7, **dadurch gekennzeichnet, dass** die Einrichtung zum Führen der Wirkstoffeinheiten eine kreisrunde Scheibe (16) aufweist an deren Umfang die Wirkstoffeinheiten (5, 14) mit Hilfe von Unterdruck (17) festgehalten werden.

**11.** Vorrichtung nach Anspruch 9 oder 10, **dadurch gekennzeichnet, dass** Einrichtungen zum Bestimmen eines Massenwertes der Trageinrichtung für die Wirkstoffeinheiten vorgesehen sind.

**12.** Vorrichtung nach einem der Ansprüche 7 bis 11 zur Bestimmung der Masse von Wirkstoffeinheiten (5, 14), die aus einer Umhüllung und dem darin enthaltenen Wirkstoff bestehen, **dadurch gekennzeichnet, dass** sie einen zweiten Mikrowellenresonator (8) mit Mess- und Auswertungselektronik zum Bestimmen der Masse der Wirkstoffeinheiten (5) vor der Befüllung aufweist.

**Claims**

**1.** Method for determining the mass of portioned active ingredient units, in particular capsules, tablets or dragées, wherein the active ingredient units are conducted through a microwave resonator and the mass M is determined from the shift A of the resonance frequency and the widening B of the resonance curve, the influence of moisture content being compensated by a mathematical linking of the two measurements, said linking consisting of a linear development from M to A, each of the coefficients representing a linear development to F, F being equal to B/A and the mass being determined by means of the relationship

$$M = k1*(1+k2*F)*A,$$

where k1 and k2 are constants and the constant k2 is dependent on the active ingredient unit investigated.

**2.** Method according to claim 1, **characterised in that** the moisture content is determined additionally.

**3.** Method according to claim 1 or 2 for determining the mass of portioned active ingredient units which consist of a shell and the active ingredient contained therein, **characterised in that** the mass of the shell without active ingredient is determined and then the total mass of the active ingredient unit after filling with the active ingredient is determined.

**4.** Method according to claim 3, **characterised in that** the empty shells are sorted by mass, are then filled in batches and the total mass is determined.

**5.** Method according to claim 3, **characterised in that** the mass of the shell is determined immediately before filling.

**6.** Method according to any one of claims 1 to 5, **characterised in that** microwaves having frequencies from 1 to 60 GHz, in particular from 2 to 30 GHz, are used.

**7.** Apparatus for determining the mass of portioned active ingredient units (5, 14), in particular capsules, tablets or dragées, comprising a microwave generator (3), a microwave resonator (1), a device (10, 15, 16) for conducting the active ingredient units (5, 14) through the microwave resonator (1), measurement and evaluation electronics (4) for determining the mass from the shift A of the resonance frequency and the widening B of the resonance curve, and a device for ejecting individual active ingredient units (5, 14).

**8.** Apparatus according to claim 7, **characterised in that** the device for conducting the active ingredient units includes a tube (10, 15) through which the active ingredient units (5, 14) are conveyed by means of an air stream (21).

**9.** Apparatus according to claim 7, **characterised in that** the device for conducting the active ingredient units includes an endless belt (18) having depressions (22) into which the active ingredient units are placed.

**10.** Apparatus according to claim 7, **characterised in that** the device for conducting the active ingredient units includes a circular plate (16) on the periphery of which the active ingredient units (5, 14) are retained by means of a partial vacuum (17).

**11.** Apparatus according to claim 9 or 10, **characterised in that** arrangements for determining a mass of the carrying device for the active ingredient units are provided.

**12.** Apparatus according to any one of claims 7 to 11 for determining the mass of active ingredient units (5, 14) consisting of a shell and the active ingredient contained therein, **characterised in that** it includes a second microwave resonator (8) having measurement and evaluation electronics for determining the mass of the active ingredient units (5) prior to filling.

**Revendications**

**1.** Procédé de détermination de la masse d'unités de principe actif prédosées, notamment des capsules, des comprimés ou des dragées, dans lequel les unités de principe actif sont guidées par un résonateur à micro-ondes et, à partir du décalage A de la fréquence de résonance et de la propagation B de la courbe de résonance, la masse M est déterminée, avec compensation de l'influence de l'humidité, grâce à une relation mathématique entre les deux grandeurs de mesure, qui consiste en un développement linéaire de M vers A, chacun des coefficients représentant un développement linéaire vers F, où F = B/A et la masse est déterminé au moyen de la relation

$$M = K1 * (1 + K2 * F) * A$$

où K1 et K2 sont des constantes, la constante K2 étant liée au principe actif étudié.

2. Procédé selon la revendication 1, **caractérisé en ce que** l'on détermine en plus l'humidité.

3. Procédé de détermination de la masse d'unités de principe actif prédosées qui sont constituées d'une enveloppe et du principe actif qu'elle contient selon la revendication 1 ou 2, **caractérisé en ce qu'**on détermine la masse de l'enveloppe sans le principe actif et ensuite la masse totale de l'unité de principe actif après remplissage avec le principe actif.

4. Procédé selon la revendication 3, **caractérisé en ce que** les enveloppes vides sont classées par masse et remplies ensuite par charges et qu'ensuite la masse totale est déterminée.

5. Procédé selon la revendication 3, **caractérisé en ce que** la masse de l'enveloppe est déterminée directement avant le remplissage.

6. Procédé selon l'une des revendications 1 à 5, **caractérisé en ce que** des micro-ondes avec une fréquence de 1 à 60 GHz, notamment de 2 à 30 GHz sont utilisées.

7. Dispositif pour la détermination de la masse d'unités de principe actif prédosés (5, 14), notamment des capsules, des comprimés ou des dragées, qui présente un générateur de (3), un résonateur de micro-ondes (1), un dispositif (10, 15, 16) pour guider les unités de principe actif (5, 14) à travers le résonateur de micro-ondes (1), une électronique de mesure et d'exploitation (4) pour déterminer la masse à partir du décalage A de la fréquence de résonance et de la propagation B de la courbe de résonance et un dispositif pour écluser au dehors chacune des unités de principe actif (5, 14).

8. Dispositif selon la revendication 7, **caractérisé en ce que** le dispositif pour guider les unités de principe actif présente un tube (10, 5), à travers lequel les unités de principe actif (5, 14) sont transportées par un flux d'air (21).

9. Dispositif selon la revendication 7, **caractérisé en ce que** le dispositif pour guider les unités de principe actif présente une bande sans fin (18) avec des évidements (22) dans lesquels les unités de principe actif sont déposées.

10. Dispositif selon la revendication 7, **caractérisé en ce que** le dispositif pour guider les unités de principe actif présente un disque circulaire (16) à la périphérie duquel les unités de principe actif (5, 14) sont maintenues au moyen d'une dépression (17).

11. Dispositif selon la revendication 9 ou 10, **caractérisé en ce qu'**il est prévu des dispositifs pour déterminer une valeur de masse du dispositif de support pour les unités de principe actif.

12. Dispositif selon l'une des revendications 7 à 11, pour la détermination de la masse d', 14), constituées d'une enveloppe et du principe actif qu'elle contient, **caractérisé en ce qu'**il présente un second résonateur de micro-ondes (8), avec une électronique de mesure et d'exploitation pour déterminer la masse des unités de principe actif (5) avant le remplissage.

**Fig. 1**

Frequenz (MHz)

**Fig. 2**

Füllgewicht der Kapseln/ mg

**Fig. 3**

**Fig. 4**

**Fig. 5**

**Fig. 6**

**Fig. 7**

**Fig. 8**

**Fig. 9**

**Fig. 10**